# EUROPÄISCHE PATENTANMELDUNG

(11) **EP 3 175 838 A1**
(43) Veröffentlichungstag der Anmeldung: **07.06.2017**
(21) Anmeldenummer: 17161238.5
(22) Anmeldetag: 16.03.2017
(51) Int. Cl.: A61K 8/44, A61K 8/46, A61Q 19/00, A61K 8/04, A61K 8/06

(54) **GESCHÄUMTE, ÖL-IN-WASSER-EMULSION ENTHALTENDE FORMULIERUNGEN ENTHALTEND MINDESTENS EIN CO-TENSID**

(71) Anmelder: Evonik Degussa GmbH, 45128 Essen (DE)
(72) Erfinder: Unger, Frank, 46147 Oberhausen (DE); Friedrich, Achim, 45529 Hattingen (DE)

(57) **Zusammenfassung**

Gegenstand der Erfindung sind geschäumte, Öl-in-Wasser-Emulsion enthaltende Formulierungen enthaltend mindestens ein Co-Tensid, sowie ein Verfahren beziehungsweise eine Vorrichtung zu ihrer Herstellung.

## Beschreibung

### Gebiet der Erfindung

Gegenstand der Erfindung sind geschäumte, Öl-in-Wasser-Emulsion enthaltende Formulierungen enthaltend mindestens ein Co-Tensid, sowie ein Verfahren beziehungsweise eine Vorrichtung zu ihrer Herstellung.

### Stand der Technik

Schäumende Öl-in-Wasser-Emulsionen sind in der kosmetischen Anwendung insbesondere bei *rinse-off-*Applikationen beschrieben, wie zum Beispiel für Wasch- und Reinigungsformulierungen. So beschreibt beispielsweise die US20060217283 schäumende Zusammensetzungen in Form von Öl-in-Wasser-Emulsionen mit bestimmter Öltröpfchengröße enthaltend ein Emulgatorsystem und weitere, schäumende, oberflächenaktive Substanzen zur Verwendung als Hautreinigungsprodukt. Es werden keine Darreichungsformen in Form von geschäumten Formulierungen beschrieben, wie sie etwa durch den Einsatz von Treibgasen entstehen, noch eine leave-on-Applikation

Aufgabe der Erfindung war es, Formulierungen bereitzustellen, die sich hervorragend als *leave-on-*Formulierungen eignen, um auf der Haut vorteilhafte Effekte zu erzielen.

### Beschreibung der Erfindung

Überraschenderweise wurde gefunden, dass die im Folgenden beschriebenen, geschäumten, ÖI-in-Wasser-Emulsion enthaltenden Formulierungen die der Erfindung gestellte Aufgabe zu lösen vermögen.

Gegenstand der vorliegenden Erfindung sind daher geschäumte, Öl-in-Wasser-Emulsion enthaltende Formulierungen enthaltend mindestens ein Co-Tensid.
Ein weiterer Gegenstand der Erfindung ist ein Verfahren zur Herstellung der geschäumten, ÖI-in-Wasser-Emulsion enthaltenden Formulierungen.
Noch ein Gegenstand der Erfindung ist eine Vorrichtung zur Herstellung der geschäumten, ÖI-in-Wasser-Emulsion enthaltenden Formulierungen.

Ein Vorteil der vorliegenden Erfindung ist es, dass die geschäumten, Öl-in-Wasser-Emulsion enthaltenden Formulierungen sich hervorragend auf der Haut verteilen lassen.

Noch ein Vorteil der vorliegenden Erfindung ist es, dass die geschäumten, Öl-in-Wasser-Emulsion enthaltenden Formulierungen eingearbeitete Inhaltsstoffe sehr gleichmäßig auf der Haut verteilen, so dass zum Beispiel Feststoffpartikel zu einem ebenmäßigen Hautbild führen.
Noch ein Vorteil der vorliegenden Erfindung ist es, dass die geschäumten, Öl-in-Wasser-Emulsion enthaltenden Formulierungen bei der Anwendung nicht tropfen.
Noch ein Vorteil der vorliegenden Erfindung ist es, dass bei der Anwendung der geschäumten, ÖI-in-Wasser-Emulsion enthaltenden Formulierungen im Gegensatz zu bekannten Aerosolsprays keine vernebelten lungengängigen Anteile erzeugt werden.
Noch ein Vorteil der vorliegenden Erfindung ist es, dass derartige geschäumte, ÖI-in-Wasser-Emulsion enthaltende Formulierungen eine vorteilhafte Textur aufweisen.
Noch ein Vorteil der vorliegenden Erfindung ist es, dass die geschäumten, Öl-in-Wasser-Emulsion enthaltenden Formulierungen beim Verteilen eine kühlende Wirkung entfalten.
Noch ein Vorteil der vorliegenden Erfindung ist es, dass aufgrund der Zusammensetzung und der Abgeschlossenheit zur Umgebung nur eine minimale Konservierung der geschäumten, ÖI-in-Wasser-Emulsion enthaltenden Formulierungen notwendig ist.

Gegenstand der vorliegenden Erfindung ist eine die geschäumte, Öl-in-Wasser-Emulsion enthaltende Formulierung enthaltend mindestens ein Co-Tensid.

Unter dem Begriff "geschäumt" wird im Zusammenhang mit der vorliegenden Erfindung eine Formulierung verstanden, die, in klarer Abgrenzung zu einer "schäumenden" Formulierung zu ihrem allergrößten Anteil in Form eines Schaumes vorliegt.
Unter dem Begriff "Schaum" wird im Zusammenhang mit der vorliegenden Erfindung eine Zusammensetzung verstanden, welche gasförmige Bläschen, die wiederum durch flüssige Wände eingeschlossen sind, enthalten. In die geschäumten, Öl-in-Wasser-Emulsion enthaltenden Formulierungen der vorliegenden Erfindung bildet die Öl-in-Wasser-Emulsion die flüssigen Wände um die gasförmigen Bläschen.
Alle angegebenen Prozent (%) sind, wenn nicht anders angegeben, Massenprozent.

Bevorzugte geschäumte, Öl-in-Wasser-Emulsion enthaltende Formulierungen der vorliegenden Erfindung sind dadurch gekennzeichnet, dass bezogen auf ihr Gesamtvolumen, welches aus im Schaum eingeschlossenen Gas und Öl-in-Wasser-Emulsion besteht, die Öl-in-Wasser-Emulsion 1,0 vol.-% bis 28 vol.-%, bevorzugt 1,3 vol.-% bis 20 vol.-%, besonders bevorzugt 1,7 vol.-% bis 12 vol.-%, ausmacht.

Bevorzugte geschäumte, Öl-in-Wasser-Emulsion enthaltende Formulierungen der vorliegenden Erfindung sind dadurch gekennzeichnet, dass 1 g der geschäumten Öl-in-Wasser-Emulsion bei 25 °C und 1 bar Druck ein Volumen von 3,5 cm³ bis 100 cm³, bevorzugt 5,0 cm³ bis 80 cm³ besonders bevorzugt 8,0 cm³ bis 60 cm³, aufweist.

Bevorzugte geschäumte, Öl-in-Wasser-Emulsion enthaltende Formulierungen der vorliegenden Erfindung sind dadurch gekennzeichnet, dass das Co-Tensid ausgewählt ist aus der Gruppe bestehend aus Betaine, Amphoacetate, Amphodiacetate und Sulfosuccinate. Bevorzugte geschäumte, Öl-in-Wasser-Emulsion enthaltende Formulierungen der vorliegenden Erfindung sind dadurch gekennzeichnet, dass die als Co-Tensid enthaltenen Betaine ausgewählt sind aus der Gruppe bestehend aus CocamidopropylBetaine, Undecylenamidopropyl Betaine und Capryl/Capramidopropyl Betaine.
Bevorzugte geschäumte, Öl-in-Wasser-Emulsion enthaltende Formulierungen der vorliegenden Erfindung sind dadurch gekennzeichnet, dass die als Co-Tensid enthaltenen Amphoacetate und Amphodiacetate ausgewählt sind aus der Gruppe bestehend aus Cocoamphoacetate, Cocoamphodiacetate sowie Lauroamphodiacetate.
Bevorzugte geschäumte, Öl-in-Wasser-Emulsion enthaltende Formulierungen der vorliegenden Erfindung sind dadurch gekennzeichnet, dass die als Co-Tensid enthaltenen Sulfosuccinate ausgewählt sind aus der Gruppe bestehend aus Diethylhexyl Sulfosuccinate, Lauryl Sulfosuccinate, Laureth Sulfosuccinate, PEG-4 Cocamido MIPA-Sulfosuccinate, PEG-5 Laurylcitrat Sulfosuccinate, Undecylenamido MEA-Sulfosuccinate sowie Ricinoleamido MEA-Sulfosuccinate.

Bevorzugte geschäumte, Öl-in-Wasser-Emulsion enthaltende Formulierungen der vorliegenden Erfindung sind dadurch gekennzeichnet, dass sie bezogen auf die Gesamtformulierung
a) 3 Gew.-% bis 75 Gew.-%, bevorzugt 5 Gew.-% bis 50 Gew.-%, besonders bevorzugt 10 Gew.-% bis 30 Gew.-% Ölphase,
b) 20 Gew.-% bis 93,5 Gew.-%, bevorzugt 40 Gew.-% bis 89,2 Gew.-%, besonders bevorzugt 60 Gew.-% bis 82,5 Gew.-% Wasserphase,
c) 0,5 Gew.-% bis 10,0 Gew.-%, bevorzugt 1,0 Gew.-% bis 7,0 Gew.-%, besonders bevorzugt 1,5 Gew.-% bis 5,0 Gew.-% Öl-in-Wasser-Emulgator,
d) 0,5 Gew.-% bis 10 Gew.-%, bevorzugt 0,8 Gew.-% bis 8,0 Gew.-%, besonders bevorzugt 1,0 Gew.-% bis 5,0 Gew.-% ein oder mehrere Co-Tenside und
e) 2,5 Gew.-% bis 50 Gew.-%, bevorzugt 4 Gew.-% bis 30 Gew.-%, besonders bevorzugt 5 Gew.-% bis 20 Gew.-% Treibgas
enthält.

Bevorzugt enthaltenes Öl ist ausgewählt aus mindestens einem der Gruppe der kosmetischen Öle. Unter dem Begriff "kosmetisches Öl" im Zusammenhang mit der vorliegenden Erfindung sind mit Wasser nicht-mischbare Flüssigkeiten, welche zur Herstellung kosmetischer Formulierungen geeignet sind, zu verstehen. Mit Wasser nicht mischbar bedeutet im Zusammenhang mit der vorliegenden Erfindung, dass bei Raumtemperatur wässrige Mischungen von kosmetischen Ölen bei Ölkonzentrationen von 0,5 - 99,5 vol.-% bezogen auf die Gesamtmischung zu einer bereits mit dem menschlichen Auge wahrnehmbaren Trübung bzw. zur Ausbildung von zwei oder mehr Phasen führen. Des Weiteren sind im Zusammenhang mit der vorliegenden Erfindung kosmetische Öle bevorzugt dadurch gekennzeichnet, dass sie zu Wasser ein Grenzflächenspannung von > 5 mN/m aufweisen. Kosmetische Öle können zum Beispiel Oleochemie- oder Silikonchemie basiert sein.
Insbesondere sind diese ausgewählt aus der Gruppe der Fettalkohole, Ester von linearen Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen, Ester von Alkylhydroxycarbonsäuren mit linearen oder verzweigten Fettalkoholen. Des Weiteren Mono-, Di- oder Triglyceride in flüssiger oder fester Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen. Des Weiteren Ether, mit oder ohne Alkoxygruppen, Silikonöle, mit oder ohne organische Modifizierung. Des Weiteren Mischungen dieser Öle in beliebigen Verhältnissen.

Erfindungsgemäß bevorzugt ausgewählte Öl-in-Wasser-Emulgatoren sind ausgewählt aus der Gruppe bestehend aus Polyglycerinester und Glycerinester, modifizierte Carbohydrate, organomodifizierte Siloxane und Ethoxylate.
Bevorzugte geschäumte, Öl-in-Wasser-Emulsion enthaltende Formulierungen der vorliegenden Erfindung sind dadurch gekennzeichnet, dass die als Öl-in-Wasser-Emulgator enthaltenen (Poly)glycerinester ausgewählt sind aus der Gruppe bestehend aus Glycerinfettsäureester und Polyglycerinfettsäureester, insbesondere Polyglycerinfettsäureester, wobei Glyceryl Stearate Citrate, Glyceryl Stearate SE, Polyglyceryl-3 Dicitrate/Stearate, Polyglyceryl-3 Distearate, Polyglyceryl-3 Methylglucose Distearate, Polyglyceryl-6 Stearate, Polyglyceryl-6 Behenate, Methyl Glucose Sesquistearate und Polyglyceryl-4 Laurate besonders bevorzugt sind.
Bevorzugte geschäumte, Öl-in-Wasser-Emulsion enthaltende Formulierungen der vorliegenden Erfindung sind dadurch gekennzeichnet, dass die als Öl-in-Wasser-Emulgator enthaltenen modifizierte Carbohydrate ausgewählt sind aus der Gruppe bestehend aus Cetearyl Glucoside, Sucrose Stearate sowie Sorbitan Laurate.

Bevorzugte geschäumte, Öl-in-Wasser-Emulsion enthaltende Formulierungen der vorliegenden Erfindung sind dadurch gekennzeichnet, dass die als Öl-in-Wasser-Emulgator enthaltenen organomodifizierte Siloxane ausgewählt sind aus der Gruppe bestehend aus Bis-PEG/PPG-16/16 PEG/PPG-16/16 Dimethicone, Bis-PEG/PPG-20/5 PEG/PPG-20/5 Dimethicone sowie Methoxy PEG/PPG-25/4 Dimethicone.

Bevorzugte geschäumte, Öl-in-Wasser-Emulsion enthaltende Formulierungen der vorliegenden Erfindung sind dadurch gekennzeichnet, dass die als Öl-in-Wasser-Emulgator enthaltenen Ethoxylate ausgewählt sind aus der Gruppe bestehend aus Fettalkoholethoxylate, Fettsäureesterethoxylate und Fettsäureethoxylate, wobei die vorgenannten Substanzen insbesondere 15 bis 120 Ethylenoxideinheiten aufweisen, wobei Ceteareth-15, Ceteareth-20, Ceteareth-25, Steareth-20, Steareth-21, PEG-40 Stearate, PEG-100 Stearate, PEG-40 Sorbitan Perisostearate, Laureth-23, Isoceteth-20, Polysorbate 60 sowie Polysorbate 80 besonders bevorzugt sind.

Die erfindungsgemäßen geschäumten, Öl-in-Wasser-Emulsion enthaltenden Formulierungen können z.B. mindestens eine zusätzliche Komponente enthalten, ausgewählt aus der Gruppe der
Co-Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Pigmente
kosmetische Wirkstoffe,
Pflegeadditive,
Überfettungsmittel,
Lösungsmittel,
UV-Filter,
Elektrolyte,
Multifunktionelle Additive,
feuchtigkeitsspendende Stoffe.

Es ist erfindungsgemäß bevorzugt, dass die erfindungsgemäßen geschäumten, ÖI-in-Wasser-Emulsion enthaltenden Formulierungen frei sind von ethoxylierten Fettsäureestern des Glycerins. Ein weiterer Gegenstand der vorliegenden Erfindung ist ein Verfahren zur Herstellung der erfindungsgemäßen geschäumten, Öl-in-Wasser-Emulsion enthaltenden Formulierung umfassend die Verfahrensschritte:
A) Bereitstellen einer Öl-in-Wasser-Emulsion enthaltend mindestens ein Co-Tensid in einem geschlossenen Gefäß aufweisend eine Düse
B) Erzeugen eines Überdruckes in dem geschlossenen Gefäß mit Hilfe eines Treibgases
C) Entlassen mindestens eines Teils der Öl-in-Wasser-Emulsion und des Treibgases durch die Düse unter Entstehen einer geschäumten Öl-in-Wasser-Emulsion.

Ein erfindungsgemäß bevorzugtes Verfahren ist dadurch gekennzeichnet, dass das Treibgas ausgewählt ist aus der Gruppe bestehend aus Luft, CO₂ N₂, N₂O, Propan, Butan, Isobutan und Dimethylether

Es ist erfindungsgemäß bevorzugt, wenn der Überdruck, gemessen bei 25 °C, in Verfahrensschritt B) auf zwischen 1,3 bar und 8,0 bar, bevorzugt auf zwischen 1,5 bar und 6,0 bar, besonders bevorzugt zwischen 2,0 bar und 4,5 bar gebracht wird.

In dem erfindungsgemäßen Verfahren werden die oben als bevorzugt genannten Co-Tenside, Öle und ÖI-in-Wasser-Emulgatoren bevorzugt eingesetzt.

Ein weiterer Gegenstand der vorliegenden Erfindung ist eine Vorrichtung zur Herstellung der erfindungsgemäßen geschäumten, Öl-in-Wasser-Emulsion enthaltenden Formulierung umfassend ein geschlossenes Gefäß aufweisend eine Düse, wobei das Gefäß eine Öl-in-Wasser-Emulsion, ein Co-Tensid und ein Treibgas unter Überdruck beherbergt.
In der erfindungsgemäßen Vorrichtung werden die oben als bevorzugt genannten Co-Tenside, Öle und ÖI-in-Wasser-Emulgatoren bevorzugt beherbergt.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
Alle Konzentrationen in den Anwendungsbeispielen sind in Gewichtsprozent angegeben. Zur Herstellung der Emulsionen wurden dem Fachmann bekannte übliche Homogenisierverfahren eingesetzt.
Die Herstellung der Emulsionen erfolgte daher typischerweise so, dass Öl- und Wasserphase auf 70 - 75°C erwärmt wurden. Anschließend wurde entweder die Ölphase in die Wassereingerührt oder Öl- und Wasserphase ohne Rühren zusammengegeben. Anschließend wurde mit einem geeigneten Homogenisator (z.B. Ultra Thurrax) für ca. 1-2 Minuten homogenisiert.
Stabilisierende Polymere (z.B. Carbomere) werden bevorzugt als Öldispersion bei Temperaturen von 50 - 60°C in die Emulsion eingerührt. Anschließend wird kurz homogenisiert.

Zugabe weiterer Inhaltsstoffe (z.B. Konservierungsmittel, Wirkstoffe) erfolgte bevorzugt bei 40°C. Falls die Rezepturen mit organischen Säuren konserviert wurden, wurde der pH-Wert der Emulsionen auf ca. 5 eingestellt. Danach erfolgt die Zugabe der Co-Tenside und wiederrum eine kurzer Homogenisierung. Im Anschluss wird die Emulsion in einer Aerosoldose oder einem vergleichbaren Behältnis mit dem unter Druck stehenden, verflüssigten Treibgas (z.B. DRIVOSOL^{®} 27D60 (n-Butan, iso-Butan, Propan), Evonik Perfomance Materials GmbH) in den zuvor beschriebenen Verhältnissen versetzt und durch Schütteln mit diesem gleichmäßig durchmischt.

### Beispiel 1: Verteilbarkeit und Gleitfähigkeit

Überraschenderweise wurde gefunden, dass mit Co-Tensid und Treibgas versetzte O/W Emulsionen lagerstabil sind. Gleichzeitig weisen derartige Formulierungen in ihrer geschäumten Form auch sensorische Vorteile hinsichtlich einer besseren Verteilbarkeit und Gleitfähigkeit auf. Die sensorischen Parameter werden mit einem geschulten Panel mittels einer objektiven Skala und einer standardisierten Vorgehensweise ermittelt.

| | T1 | T1-V1 | T1-V2 |
|---|---|---|---|
| Bis-PEG/PPG-20/5 PEG/PPG-20/5 Dimethicone; Methoxy PEG/PPG-25/4 Dimethicone; Carpylic/Capric Triglyceride¹ | 2,5 | 2,5 | 2,5 |
| Diethylhexyl Carbonate⁵ | 4,0 | 4,0 | 4,0 |
| Caprylic/Capric Triglyceride | 3,0 | 3,0 | 3,0 |
| Cyclopentasiloxane | 5,0 | 5,0 | 5,0 |
| Cetyl Ricinoleate¹⁹ | 1,0 | 1,0 | 1,0 |
| Wasser | Ad 100 | Ad 100 | Ad 100 |
| Glycerin | 2,0 | 2,0 | 2,0 |
| Panthenol | 0,5 | 0,5 | 0,5 |
| Allantoin | 0,2 | 0,2 | 0,2 |
| Ceramide NP; Ceramide AP; Ceramide EOP; Phytosphingosine; Cholesterol; Sodium Lauroyl Lactylate; Carbomer; Xanthan Gum²⁰ | 1,0 | 1,0 | 1,0 |
| Creatine²¹ | 0,5 | 0,5 | 0,5 |
| Carbomer ²² | 0,3 | 0,3 | 0,3 |
| Xanthan Gum | 0,1 | 0,1 | 0,1 |
| Ethylhexyl Stearate | 1,6 | 1,6 | 1,6 |
| Sodium Hydroxide (10% aq.) | 0,7 | 0,7 | 0,7 |
| Phenoxyethanol, Ethylhexylglycerin¹⁸ | 0,7 | 0,7 | 0,7 |
| Sodium Cocoamphoacetate¹⁰ | 1,0 | 1,0 | |
| Capryl/Capramidopropyl Betaine⁷ | 1,0 | 1,0 | |
| Treibgas | 10,0 | | |
| | | | |
| Stabilität | stabil | stabil | stabil |
| Verteilbarkeit | ++ | + | O |
| Gleitfähigkeit | ++ | O | + |

### Formulierungsbeispiele

Die folgenden Formulierungsbeispiele werden entsprechend der beschriebenen und dem Experten bekannten Verfahren hergestellt. Zusätzlich zu den dargestellten Inhaltsstoffen der O/W-Emulsion wird im Anschluss Treibgas (z.B. DRIVOSOL^{®} 27D60 (n-Butan, iso-Butan, Propan), Evonik Perfomance Materials GmbH) in den bereits beschriebenen Mengen in einem geeigneten Gefäß unter Druck zugegeben.

### Hautpflegeschaum

| | 1 | 2 | 3 | 4 | 5 | 6 | 7 |
|---|---|---|---|---|---|---|---|
| Bis-PEG/PPG-20/5 PEG/PPG-20/5 Dimethicone; Methoxy PEG/PPG-25/4 Dimethicone; Carpylic/Capric Triglyceride¹ | 5,0 | | | | 5,0 | 5,0 | 5,0 |
| Bis-PEG/PPG-16/16 PEG/PPG-16/16 Dimethicone; Caprylic/Capric Triglyceride² | | | | 5,0 | | | |
| Sorbitan Laurate; Polyglyceryl-4 Laurate; Dilauryl Citrate⁴ | | | 5,0 | | | | |
| Diethylhexyl Carbonate⁵ | 24,5 | 24,5 | 24,5 | 24,5 | 24,5 | 50,0 | 50,0 |
| Bis-PEG/PPG-20/20 Dimethicone⁶ | 0,5 | 0,5 | 0,5 | 0,5 | 0,5 | 0,5 | |
| Wasser | ad 100 | ad 100 | ad 100 | ad 100 | ad 100 | ad 100 | ad 100 |
| Capryl/Capramidoprop yl Betaine⁷ | 1,0 | 1,0 | 1,0 | 1,0 | 1,0 | 1,0 | 1,0 |
| Disodium Laureth Sulfosuccinate⁸ | | | | 1,0 | 1,0 | 1,0 | 1,0 |

| | | | | | | | |
|---|---|---|---|---|---|---|---|
| ¹: ABIL^{®} Care XL 80 (Evonik Nutrition & Care GmbH) ²: ABIL^{®} Care 85 (Evonik Nutrition & Care GmbH) ³: TEGO^{®} Care PBS 6 (Evonik Nutrition & Care GmbH) ⁴: TEGO^{®} Care LTP (Evonik Nutrition & Care GmbH) ⁵: TEGOSOFT^{®} DEC (Evonik Nutrition & Care GmbH) ⁶: ABIL^{®} B 8832 (Evonik Nutrition & Care GmbH) ⁷: TEGO^{®} Betain 810 (Evonik Nutrition & Care GmbH) ⁸: REWOPOL^{®} SB FA 30 B (Evonik Nutrition & Care GmbH) | | | | | | | |

### Hautpflegeschaum

| | 8 | 9 | 10 | 11 | 12 |
|---|---|---|---|---|---|
| Bis-PEG/PPG-20/5 PEG/PPG-20/5 Dimethicone; Methoxy PEG/PPG-25/4 Dimethicone; Carpylic/Capric Triglyceride¹ | 5,0 | 5,0 | | | 5,0 |
| Sorbitan Laurate; Polyglyceryl-4 Laurate; Dilauryl Citrate⁴ | | | 5,0 | | |
| Polyglyceryl-6 Stearate; Polyglyceryl-6 Behenate³ | | | | 5,0 | |
| Hydroxypropyl Methylcellulose | | 0,5 | | | |
| Xanthan Gum | | | | | 0,3 |
| Diethylhexyl Carbonate⁵ | 24,5 | 24,5 | 24,5 | 24,5 | 24,5 |
| Bis-PEG/PPG-20/20 Dimethicone⁶ | 0,5 | 0,5 | 0,5 | 0,5 | 0,5 |
| Wasser | ad 100 | ad 100 | ad 100 | ad 100 | ad 100 |
| Cocamidopropyl Betaine⁹ | 1,0 | 1,0 | 1,0 | 1,0 | 1,0 |
| Sodium Cocoamphoacetate¹⁰ | 1,0 | 1,0 | 1,0 | 1,0 | 1,0 |

| | | | | | |
|---|---|---|---|---|---|
| ⁹: TEGO^{®} Betain P 50 C (Evonik Nutrition & Care GmbH) ¹⁰: REWOTERIC^{®} AM C (Evonik Nutrition & Care GmbH) | | | | | |

### Schaum mit leichtem Hautgefühl

| | 13 | 14 | 15 |
|---|---|---|---|
| Glyceryl Stearate; PEG-100 Stearate | 6,0 | | |
| Bis-PEG/PPG-20/5 PEG/PPG-20/5 Dimethicone; Methoxy PEG/PPG-25/4 Dimethicone; Carpylic/Capric Triglyceride¹ | | | 5,0 |
| Ceteareth-25 | | 5,0 | |
| Diethylhexyl Carbonate⁵ | | | 9,5 |
| Oleyl Erucate¹¹ | 10,0 | | |
| Isoamyl Cocoate¹² | | 10,0 | |
| Caprylic/Capric Triglyceride | 15,0 | 5,0 | |
| Cyclopentasiloxane | | | 15,0 |
| Bis-PEG/PPG-20/20 Dimethicone⁶ | | | 0,5 |
| Wasser | ad 100 | ad 100 | ad 100 |
| Capryl/Capramidopro pyl Betaine⁷ | 1,0 | 1,0 | 1,0 |
| Sodium Cocoamphoacetate¹⁰ | 1,0 | 1,0 | 1,0 |

| | | | |
|---|---|---|---|
| ¹¹: TEGOSOFT^{®} OER (Evonik Nutrition & Care GmbH) ¹²: TEGOSOFT^{®} AC (Evonik Nutrition & Care GmbH) | | | |

### Hautpflegeschaum mit unterschiedlichen kosmetischen Ölen

| | 16 | 17 | 18 | 19 | 20 | 21 |
|---|---|---|---|---|---|---|
| Cetearyl Glucoside¹³ | 2,0 | 2,0 | 2,0 | 2,0 | 2,0 | 2,0 |
| Diethylhexyl Carbonate⁵ | 19,5 | | | | | |
| Isopropyl Palmitate | | 19,5 | | | | |
| Phenoxyethyl Caprylate¹⁴ | | | 19,5 | | | |
| Ethylhexyl Palmitate | | | | 19,5 | | |
| Caprylic/Capric Triglyceride | | | | | 19,5 | |
| Oleyl Erucate¹¹ | | | | | | 19,5 |
| Bis-PEG/PPG-20/20 Dimethicone⁶ | 0,5 | 0,5 | 0,5 | 0,5 | 0,5 | 0,5 |
| Wasser | ad 100 | ad 100 | ad 100 | ad 100 | ad 100 | ad 100 |
| Capryl/Capramidopro pyl Betaine⁷ | 1,0 | 1,0 | 1,0 | 1,0 | 1,0 | 1,0 |
| Sodium Cocoamphoacetate¹⁰ | 1,0 | 1,0 | 1,0 | 1,0 | 1,0 | 1,0 |

| | | | | | | |
|---|---|---|---|---|---|---|
| ¹³: TEGO^{®} Care CG 90 (Evonik Nutrition & Care GmbH) ¹⁴: TEGOSOFT^{®} XC (Evonik Nutrition & Care GmbH) | | | | | | |

### Sonnenschutzschaum SPF 30 UVA

| | 22 |
|---|---|
| Polyglyceryl-6 Stearate; Polyglyceryl-6 Behenate³ | 3,0 |
| Glyceryl Stearate | 0,5 |
| Cetearyl Alcohol | 0,5 |
| Phenoxyethyl Caprylate¹⁴ | 3,0 |
| Isopropyl Palmitate | 2,0 |
| Bis-Ethylhexyloxyphenol Methoxyphenyl Triazine¹⁵ | 3,0 |
| Butyl Methoxydibenzoylmethane | 2,0 |
| Ethylhexyl Salicylate | 3,0 |
| Octocrylene | 4,0 |
| Homosalate | 3,0 |
| Glycerin | 3,0 |
| Wasser | ad 100 |
| Acrylates/C10-30 Alkyl Acrylate Crosspolymer¹⁶ | 0,2 |
| Phenoxyethyl Caprylate¹⁴ | 0,8 |
| Tromethamine (30% aq.) | 0,6 |
| UV-Filterlösung¹⁷ | 10,0 |
| Capryl/Capramidopropyl Betaine⁷ | 1,0 |
| Sodium Cocoamphoacetate¹⁰ | 1,0 |
| Phenoxyethanol, Ethylhexylglycerin¹⁸ | 0,7 |

| | |
|---|---|
| ¹⁵: Tinosorb S (BASF SE) ¹⁶: TEGO^{®} Carbomer 341 ER (Evonik Nutrition & Care GmbH) ¹⁷ : 20 % Phenylbenzimidazole Sulfonic Acid; 8,8% Tromethamine; Demineralized Water ad 100% ¹⁸: Euxyl PE 9010 (Schülke & Mayr GmbH) | |

### Geschäumter Selbstbräuner

| | 23 |
|---|---|
| Polyglyceryl-6 Stearate; Polyglyceryl-6 Behenate³ | 3,0 |
| Glyceryl Stearate | 2,5 |
| Cetearyl Alcohol | 1,0 |
| Oleyl Erucate¹¹ | 2,0 |
| Caprylic/Capric Triglyceride | 3,0 |
| Diethylhexyl Carbonate⁵ | 3,0 |
| Isopropyl Palmitate | 3,0 |
| Glycerin | 3,0 |
| Wasser | ad 100 |
| Xanthan Gum | 0,3 |
| Dihydroxyacetone | 3,5 |
| Erythrulose | 1,5 |
| Phenoxyethanol, Ethylhexylglycerin¹⁸ | 0,7 |
| Capryl/Capramidopropyl Betaine⁷ | 1,0 |
| Sodium Cocoamphoacetate¹⁰ | 1,0 |

### After Shave Schaum

| | 24 | 25 |
|---|---|---|
| Bis-PEG/PPG-20/5 PEG/PPG-20/5 Dimethicone; Methoxy PEG/PPG-25/4 Dimethicone; Carpylic/Capric Triglyceride¹ | 2,5 | |
| Polyglyceryl-6 Stearate; Polyglyceryl-6 Behenate³ | | 3,0 |
| Glyceryl Stearate | | 0,5 |
| Cetearyl Alkohol | | 1,0 |
| Diethylhexyl Carbonate⁵ | 4,0 | 3,0 |
| Caprylic/Capric Triglyceride | 3,0 | 4,5 |
| Cyclopentasiloxane | 5,0 | 5,5 |
| Cetyl Ricinoleate¹⁹ | 1,0 | |
| Wasser | ad 100 | Ad 100 |
| Glycerin | 2,0 | 2,0 |
| Panthenol | 0,5 | 0,5 |
| Allantoin | 0,2 | 0,2 |
| Ceramide NP; Ceramide AP; Ceramide EOP; Phytosphingosine; Cholesterol; Sodium Lauroyl Lactylate; Carbomer; Xanthan Gum²⁰ | 1,0 | 1,0 |
| Creatine²¹ | 0,5 | 0,5 |
| Carbomer ²² | 0,3 | 0,25 |
| Xanthan Gum | 0,1 | |
| Ethylhexyl Stearate | 1,6 | |
| Sodium Hydroxide (10% aq.) | 0,7 | 0,75 |
| Phenoxyethanol, Ethylhexylglycerin ¹⁸ | 0,7 | 0,7 |
| Capryl/Capramidopropyl Betaine⁷ | 1,0 | 1,0 |
| Sodium Cocoamphoacetate¹⁰ | 1,0 | 1,0 |

| | | |
|---|---|---|
| ¹⁹: TEGOSOFT^{®} CR (Evonik Nutrition & Care GmbH) ²⁰: SK-INFLUX^{®} V (Evonik Nutrition & Care GmbH) ²¹: TEGO^{®} Cosmo C 100 (Evonik Nutrition & Care GmbH) ²²: TEGO^{®} Carbomer 141 (Evonik Nutrition & Care GmbH) | | |

### After Sun Schaum

| | 26 |
|---|---|
| Glyceryl Stearate | 0,5 |
| Stearic Acid | 0,5 |
| Isoamyl Cocoate¹² | 5,0 |
| Diethylhexyl Carbonate⁵ | 5,3 |
| Stearyl Heptanoate²² | 1,0 |
| Myristyl Myristate | 2,0 |
| Tocopheryl Acetate | 0,5 |
| Salicyloyl Phytosphingosine²⁴ | 0,2 |
| Cetearyl Glucoside¹³ | 1,0 |
| Sucrose Stearate²⁵ | 2,0 |
| Glycerin | 5,0 |
| Wasser | ad 100 |
| Panthenol | 0,5 |
| Creatine²¹ | 0,5 |
| Xanthan Gum | 0,5 |
| Ethanol | 3,0 |
| Phenoxyethanol, Ethylhexylglycerin¹⁸ | 0,7 |
| Capryl/Capramidopropyl Betaine⁷ | 1,0 |
| Sodium Cocoamphoacetate¹⁰ | 1,0 |

| | |
|---|---|
| ²³: TEGOSOFT^{®} SH (Evonik Nutrition & Care GmbH) ²⁴: Phytosphingosine SLC (Evonik Nutrition & Care GmbH) ²⁵: TEGO^{®} Care SE 121 (Evonik Nutrition & Care GmbH) | |

### Feuchtigkeitsspendender Gesichtsschaum mit natürlichen Inhaltsstoffen

| | 27 | 28 |
|---|---|---|
| Polyglyceryl-6 Stearate; Polyglyceryl-6 Behenate³ | 3,0 | 3,0 |
| Glyceryl Stearate | 1,0 | 2,0 |
| Cetearyl Alcohol | 1,0 | |
| Stearyl Alcohol | | 2,0 |
| Oleyl Erucate¹¹ | 5,0 | 5,0 |
| Decyl Cocoate | 5,0 | 5,0 |
| Myristyl Myristate | 2,0 | 3,0 |
| Avocadoöl | 3,0 | 3,0 |
| Glycerin | 3,0 | 3,0 |
| Wasser | ad 100 | ad 100 |
| Creatine²¹ | 0,5 | 0,5 |
| Betaine²⁶ | 5,0 | 5,0 |
| Xanthan Gum | 0,3 | |
| Benzyl Alcohol, Benzoic Acid, Sorbic Acid ²⁷ | 1,0 | 1,0 |
| Capryl/Capramidopropyl Betaine⁷ | 1,0 | 1,0 |
| Sodium Cocoamphoacetate¹⁰ | 1,0 | 1,0 |

| | | |
|---|---|---|
| ²⁶: TEGO^{®} Natural Betaine (Evonik Nutrition & Care GmbH) ²⁷: Rokonsal BSB-N (Ashland Specialty Ingredients) | | |

### Body Soufflé

| | 29 | 30 |
|---|---|---|
| Polyglyceryl-6 Stearate; Polyglyceryl-6 Behenate³ | 4,0 | 4,0 |
| Glyceryl Stearate | 0,5 | 2,0 |
| Stearyl Alcohol | 0,5 | 2,0 |
| Decyl Cocoate | 5,0 | 4,0 |
| Oleyl Erucate¹¹ | 5,0 | 4,0 |
| Myristyl Myristate | 1,0 | 1,0 |
| Cetyl Ricinoleate¹⁹ | 1,0 | 1,0 |
| Avocadoöl | 2,0 | 2,0 |
| Sheabutter | 6,0 | 5,0 |
| Kakaobutter | 5,0 | 5,0 |
| Glycerin | 5,0 | 5,0 |
| Wasser | ad 100 | ad 100 |
| Xanthan Gum | | 0,5 |
| Sodium Hydroxide (10% aq.) | 0,2 | 0,2 |
| Benzyl Alcohol, Benzoic Acid, Sorbic Acid ²⁷ | 0,8 | 0,8 |
| Capryl/Capramidopropyl Betaine⁷ | 1,0 | 1,0 |
| Sodium Cocoamphoacetate¹⁰ | 1,0 | 1,0 |

### Body Deo Schaum

| | 31 | 32 |
|---|---|---|
| Polyglyceryl-6 Stearate; Polyglyceryl-6 Behenate; C18-22 Hydroxyalkyl Hydroxypropyl Guar²⁸ | 4,0 | 4,0 |
| Glyceryl Stearate | | 0,5 |
| Isoamyl Cocoate¹² | 4,0 | 4,0 |
| Caprylic/Capric Triglyceride | 5,5 | 5,5 |
| Polyglyceryl-3 Caprylate²⁹ | 0,5 | 0,5 |
| Glycerin | 5,0 | 5,0 |
| Wasser | ad 100 | ad 100 |
| Sodium Benzoate, Potassium Sorbate³⁰ | 0,5 | 0,5 |
| Capryl/Capramidopropyl Betaine⁷ | 0,5 | 0,5 |
| Sodium Cocoamphoacetate¹⁰ | 0,5 | 0,5 |
| Parfum | 1,0 | 1,0 |
| Zitronensäure (50% aq.) | pH=5-5,5 | pH=5-5,5 |

| | | |
|---|---|---|
| ²⁸: TEGO® Care APD 18 (Evonik Nutrition & Care GmbH) ²⁹: TEGO^{®} Cosmo P 813 (Evonik Nutrition & Care GmbH) ³⁰: Euxyl K 712 (Schülke & Mayr GmbH) | | |

### Cooling After Sun Schaum

| | 33 |
|---|---|
| Polyglyceryl-6 Stearate; Polyglyceryl-6 Behenate³ | 4,0 |
| Glyceryl Stearate | 0,5 |
| Cetearyl Alcohol | 0,5 |
| Isoamyl Cocoate¹² | 6,0 |
| Caprylic/Capric Triglyceride | 5,3 |
| Stearyl Heptanoate²² | 2,0 |
| Tocopheryl Acetate | 0,5 |
| Salicyloyl Phytosphingosine²⁴ | 0,2 |
| Cellulose³¹ | 1,0 |
| Glycerin | 3,0 |
| Wasser | ad 100 |
| Panthenol | 0,5 |
| Creatine²¹ | 0,5 |
| Xanthan Gum | 0,2 |
| Carbomer ²² | 0,2 |
| Ethylhexyl Palmitate | 0,8 |
| Sodium Hydroxide (10% aq.) | 0,6 |
| Phenoxyethanol, Ethylhexylglycerin¹⁸ | 0,7 |
| Ethanol | 10 |
| Capryl/Capramidopropyl Betaine⁷ | 1,0 |
| Sodium Cocoamphoacetate¹⁰ | 1,0 |

| | |
|---|---|
| ³¹: TEGO^{®} Feel C 10 (Evonik Nutrition & Care GmbH) | |

### Massage Schaum

| | 34 | 35 |
|---|---|---|
| Polyglyceryl-6 Stearate; Polyglyceryl-6 Behenate³ | 4,0 | 4,0 |
| Decyl Cocoate | 66,0 | 30,0 |
| Oleyl Erucate¹¹ | | 10,0 |
| Cetyl Ricinoleate¹⁹ | | 3,0 |
| Myristyl Myristate | | 3,0 |
| Mandelöl | | 20,0 |
| Glycerin | 2,0 | 7,0 |
| Wasser | ad 100 | ad 100 |
| Benzyl Alcohol, Benzoic Acid, Sorbic Acid ²⁷ | 0,8 | 0,8 |
| Sodium Cocoamphoacetate¹⁰ | 1,0 | 1,0 |
| Capryl/Capramidopropyl Betaine⁷ | 1,0 | 1,0 |
| Zitronensäure (50% aq.) | pH= 5-5,5 | pH= 5-5,5 |

### Ice Ball

| | 36 | 37 | 38 | 39 |
|---|---|---|---|---|
| Bis-PEG/PPG-20/5 PEG/PPG-20/5 Dimethicone; Methoxy PEG/PPG-25/4 Dimethicone; Carpylic/Capric Triglyceride¹ | 3,0 | 3,0 | 3,0 | 2,5 |
| Cyclopentasiloxane | | 5,0 | | |
| Myristyl Myristate | 2,0 | 4,0 | 2,0 | |
| Stearyl Heptanoate²² | 10,0 | 14,0 | 13,0 | 11,5 |
| Diethylhexyl Carbonate⁵ | 5,0 | | | |
| Menthol | | 2,0 | 2,0 | 1,0 |
| Wasser | ad 100 | ad 100 | ad 100 | ad 100 |
| Propylenglycol | 2,0 | 2,0 | 2,0 | 2,0 |
| Carbomer ²² | 0,2 | 0,2 | 0,2 | 0,2 |
| Ethylhexyl Stearate | 0,8 | 0,8 | 0,8 | 0,8 |
| Sodium Hydroxide (10% aq.) | 0,6 | 0,6 | 0,6 | 0,6 |
| Xanthan Gum | 0,5 | 0,5 | 0,5 | 0,5 |
| Phenoxyethanol, Ethylhexylglycerin¹⁸ | 0,7 | 0,7 | 0,7 | 0,7 |
| Capryl/Capramidopropyl Betaine⁷ | 1,0 | 1,0 | 1,0 | 1,0 |
| Sodium Cocoamphoacetate¹⁰ | 1,0 | 1,0 | 1,0 | 1,0 |

### Winter Guard Schaum

| | 40 |
|---|---|
| Polyglyceryl-6 Stearate; Polyglyceryl-6 Behenate³ | 3,0 |
| Glyceryl Stearate | 0,5 |
| Cetearyl Alcohol | 0,5 |
| Petrolatum | 10,0 |
| Mineral Oil | 8,5 |
| Cetyl Ricinoleate¹⁹ | 2,0 |
| Triacetin | 0,5 |
| Glycerin | 10,0 |
| Wasser | ad 100 |
| Xanthan Gum | 0,5 |
| Harnstoff | 5,0 |
| Phenoxyethanol, Ethylhexylglycerin¹⁸ | 0,7 |
| Sodium Cocoamphoacetate¹⁰ | 1,0 |
| Capryl/Capramidopropyl Betaine⁷ | 1,0 |

### Skin Refiner Foam

| | 41 | 42 |
|---|---|---|
| Bis-PEG/PPG-20/5 PEG/PPG-20/5 Dimethicone; Methoxy PEG/PPG-25/4 Dimethicone; Carpylic/Capric Triglyceride¹ | 2,5 | 2,5 |
| Isoamyl Cocoate¹² | 9,5 | 9,5 |
| Cetyl Ricinoleate¹⁹ | 2,0 | 2,0 |
| Polyisobutene³² | 0,5 | 0,5 |
| Titanium Dioxide; Mica; Tin Oxide³³ | 0,5 | 2,0 |
| Wasser | ad 100 | ad 100 |
| Glycerin | 3,0 | 3,0 |
| Carbomer²² | 0,2 | 0,2 |
| Ethylhexyl Palmitate | 0,8 | 0,8 |
| Xanthan Gum | 0,3 | 0,3 |
| Sodium Hydroxide (10% aq.) | 0,6 | 0,6 |
| Phenoxyethanol, Ethylhexylglycerin¹⁸ | 0,7 | 0,7 |
| Capryl/Capramidopropyl Betaine⁷ | 1,0 | 1,0 |
| Sodium Cocoamphoacetate¹⁰ | 1,0 | 1,0 |

| | | |
|---|---|---|
| ³²: REWOPAL^{®} PIB 1000 (Evonik Nutrition & Care GmbH) ³³: RonaFlair Balance Gold (Merck KGaA) | | |

### After Sun Schaum

| | 43 | 44 | 45 | 46 |
|---|---|---|---|---|
| Glyceryl Stearate | 0,5 | 0,5 | 0,5 | 0,5 |
| Stearic Acid | 0,5 | 0,5 | | |
| Glyceryl Stearate Citrate | | 1,5 | | |
| Glyceryl Stearate SE | | 1,5 | 1,5 | 1,5 |
| Steareth-20 | | | 3,0 | |
| Polysorbate 80 | | | | 3,0 |
| Decyl Cocoate | 5,0 | 5,0 | 5,0 | 5,0 |
| Diethylhexyl Carbonate⁵ | 5,3 | 5,3 | 5,3 | 5,3 |
| Stearyl Heptanoate²² | 1,0 | 1,0 | 1,0 | 1,0 |
| Myristyl Myristate | 2,0 | 2,0 | 2,0 | 2,0 |
| Tocopheryl Acetate | 0,5 | 0,5 | 0,5 | 0,5 |
| Salicyloyl Phytosphingosine²⁴ | 0,2 | 0,2 | 0,2 | 0,2 |
| Cetearyl Glucoside¹³ | 1,5 | | | |
| Glycerin | 5,0 | 5,0 | 5,0 | 5,0 |
| Wasser | ad 100 | ad 100 | ad 100 | ad 100 |
| Panthenol | 0,5 | 0,5 | 0,5 | 0,5 |
| Creatine²¹ | 0,5 | 0,5 | 0,5 | 0,5 |
| Xanthan Gum | 0,5 | 0,5 | 0,5 | 0,5 |
| Ethanol | 3,0 | 5,0 | 5,0 | 5,0 |
| Phenoxyethanol, Ethylhexylglycerin¹⁸ | 0,7 | 0,7 | 0,7 | 0,7 |
| Capryl/Capramidopropyl Betaine⁷ | 1,0 | 1,0 | 1,0 | 1,0 |
| Sodium Cocoamphoacetate¹⁰ | 1,0 | 1,0 | 1,0 | 1,0 |

### Sonnenschutzschaum SPF 50

| | 47 | 48 | 49 | 50 | 51 | 52 | 53 | 54 |
|---|---|---|---|---|---|---|---|---|
| Polyglyceryl-6 Stearate; Polyglyceryl-6 Behenate³ | 3,0 | 3,0 | 3,0 | 3,0 | 3,0 | 3,0 | 3,0 | 3,0 |
| C12-15 Alkyl Benzoate | 8,0 | 5,0 | 6,0 | 4,0 | 4,0 | | | 5,0 |
| Phenoxyethyl Caprylate¹⁴ | | | | | | 4,0 | 8,0 | |
| Bis-Ethylhexyloxyphenol Methoxyphenyl Triazine¹⁵ | 4,5 | 4,5 | 6,0 | 4,5 | 4,5 | 4,5 | 4,5 | 4,5 |
| Butyl Methoxydibenzoylmethane | | | | 3,0 | 3,0 | 3,0 | | |
| Octocrylene | | | | 8,0 | 8,0 | 8,0 | | |
| Homosalate | | 5,0 | 2,0 | 6,0 | 6,0 | 6,0 | | 5,0 |
| Ethylhexyl Salicylate | | 5,0 | 2,0 | 2,5 | 2,5 | 2,5 | | 5,0 |
| Ethylhexyl Methoxycinnamate | | 2,0 | | 1,0 | 1,0 | 1,0 | | 2,0 |
| Diethylamino Hydroxybenzoyl Hexyl Benzoate³⁴ | 5,0 | 5,0 | 4,0 | | | | 5,0 | 5,0 |
| Ethylhexyl Triazone³⁵ | 3,0 | | 2,0 | | | | 3,0 | |
| Cellulose³¹ | 2,0 | 2,0 | 2,0 | 2,0 | 2,0 | | | |
| Ethyl Butylacetylaminopropionate³⁶ | | | | | | 3,5 | | |
| Bis-PEG/PPG-20/20 Dimethicone⁶ | | | | | | | 0,5 | |
| Sorbitan Oleate | | | | | | | | 0,5 |
| Wasser | ad 100 | ad 100 | ad 100 | ad 100 | ad 100 | Ad 100 | Ad 100 | Ad 100 |
| Glycerin | 2,0 | 2,0 | 2,0 | 2,0 | 2,0 | 2,0 | 2,0 | 3,0 |
| Carbomer ²² | 0,2 | 0,2 | 0,2 | 0,2 | 0,2 | 0,2 | 0,2 | 0,2 |
| C12-15 Alkyl Benzoate | 0,8 | 0,8 | 0,8 | 0,8 | 0,8 | 0,8 | 0,8 | 0,8 |
| Xanthan Gum | 0,2 | 0,2 | 0,2 | 0,2 | 0,2 | 0,2 | 0,2 | 0,2 |
| Tromethamine (30% aq.) | 0,6 | 0,6 | 0,6 | 0,6 | 0,6 | 0,6 | 0,6 | 0,6 |
| UV-Filterlösung¹⁷ | 20,0 | 20,0 | 20,0 | 15,0 | 15,0 | 15,0 | 20,0 | 20,0 |
| Phenoxyethanol, Ethylhexylglycerin¹⁸ | 0,7 | 0,7 | 0,7 | 0,7 | 0,7 | 0,7 | 0,7 | 0,7 |
| Capryl/Capramidopropyl Betaine⁷ | 1,0 | 1,0 | 1,0 | 1,0 | | 1,0 | 1,0 | 1,0 |
| Sodium Cocoamphoacetate¹⁰ | 1,0 | 1,0 | 1,0 | | 1,0 | 1,0 | 1,0 | 1,0 |

| | | | | | | | | |
|---|---|---|---|---|---|---|---|---|
| ³⁴: Uvinul A Plus (BASF SE) ³⁵: Uvinul T 150 (BASF SE) ³⁶: Insect repellent IR 3535 (Merck KGaA) | | | | | | | | |

### Wash off Carbon Mask / Detox

| | 55 | 56 |
|---|---|---|
| Polyglyceryl-6 Stearate; Polyglyceryl-6 Behenate³ | 3,0 | 3,0 |
| Cetearyl Alcohol | 0,5 | 0,5 |
| Glyceryl Stearate | 0,5 | 0,5 |
| Phenoxyethyl Caprylate¹⁴ | 8,0 | 8,0 |
| Oleyl Erucate¹¹ | 4,0 | 4,0 |
| Decyl Cocoate | 4,0 | 3,8 |
| Cetyl Ricinoleate¹⁹ | 2,0 | 2,0 |
| Salicyloyl Phytosphingosine²⁴ | | 0,2 |
| Cl 77499; Sodium Glycerophosphate³⁷ | 0,5 | 0,5 |
| Wasser | ad 100 | ad 100 |
| Glycerin; Water; Tetrapeptide-63 Acetate³⁸ | | 2,0 |
| Glycerin | 2,0 | 3,0 |
| Xanthan Gum | 0,5 | 0,5 |
| Aktivkohle | 1,0 | 1,0 |
| Benzyl Alcohol, Benzoic Acid, Sorbic Acid ²⁷ | 1,0 | 1,0 |
| Sodium Cocoamphoacetate¹⁰ | 2,0 | 2,0 |
| Cocamidopropyl Betaine³⁹ | 2,0 | 2,0 |

| | | |
|---|---|---|
| ³⁷: Unipure Black LC 989 SGP (Sensient Technologies) ³⁸: TEGO^{®} Pep 4-Comfort (Evonik Nutrition & Care GmbH) ³⁹: TEGO^{®} Betain F 50 (Evonik Nutrition & Care GmbH) | | |

### Foam to Make-up

| | 57 |
|---|---|
| Polyglyceryl-6 Stearate; Polyglyceryl-6 Behenate³ | 3,0 |
| Cetearyl Alcohol | 0,5 |
| Glyceryl Stearate | 0,5 |
| Diethylhexyl Carbonate⁵ | 7,5 |
| Cetyl Ricinoleate¹⁹ | 3,0 |
| Polyisobutene³² | 0,,5 |
| Glycerin | 3,0 |
| Wasser | add 100 |
| Xanthan Gum | 0,3 |
| Phenoxyethanol, Ethylhexylglycerin¹⁸ | 0,7 |
| Sodium Cocoamphoacetate¹⁰ | 1,0 |
| Capryl/Capramidopropyl Betaine⁷ | 1,0 |
| Cl 77499; Cl 77492; Cl 77491; Titanium Dioxide; Isopropyl Myristate; Triethyl Citrate; Acrylates/Ammonium Methacrylate Copolymer⁴⁰ | 5,0 |

| | |
|---|---|
| ⁴⁰: TagraCap3 Brown (Tagra Biotechnologies Ltd.) | |

### Antiperspirant Foam

| | 58 |
|---|---|
| Polyglyceryl-6 Stearate; Polyglyceryl-6 Behenate; C18-22 Hydroxyalkyl Hydroxypropyl Guar²⁸ | 4,0 |
| Isopropyl Palmitate | 6,0 |
| Wasser | add 100 |
| Glycerin | 1,0 |
| Aluminum Chlorohydrate (50% aq.) | 20,0 |
| Capryl/Capramidopropyl Betaine⁷ | 1,0 |
| Sodium Cocoamphoacetate¹⁰ | 1,0 |

## Patentansprüche

1. Geschäumte, Öl-in-Wasser-Emulsion enthaltende Formulierung enthaltend mindestens ein Co-Tensid.

2. Geschäumte, Öl-in-Wasser-Emulsion enthaltende Formulierung gemäß Anspruch 1, **dadurch gekennzeichnet, dass** bezogen auf ihr Gesamtvolumen, welches aus im Schaum eingeschlossenen Gas und Öl-in-Wasser-Emulsion besteht, die Öl-in-Wasser-Emulsion 1,0 vol.-% bis 28 vol.-%, bevorzugt 1,3 vol.-% bis 20 vol.-%, besonders bevorzugt 1,7 vol.-% bis 12 vol.-%, ausmacht.

3. Geschäumte, Öl-in-Wasser-Emulsion enthaltende Formulierung gemäß Anspruch 1 oder 2, **dadurch gekennzeichnet, dass** 1 g der geschäumten Öl-in-Wasser-Emulsion enthaltenden Formulierung bei 25 °C und 1 bar Druck ein Volumen von 3,5 cm³ bis 100 cm³, bevorzugt 5,0 cm³ bis 80 cm³ besonders bevorzugt 8,0 cm³ bis 60 cm³, aufweist.

4. Geschäumte, Öl-in-Wasser-Emulsion enthaltende Formulierung gemäß mindestens einem der vorherigen Ansprüche, **dadurch gekennzeichnet, dass** das Co-Tensid ausgewählt ist aus der Gruppe bestehend aus bestehend aus Betaine, Amphoacetate, Amphodiacetate und Sulfosuccinate.

5. Geschäumte, Öl-in-Wasser-Emulsion enthaltende Formulierung gemäß mindestens einem der vorherigen Ansprüche, **dadurch gekennzeichnet, dass** sie bezogen auf die Gesamtformulierung
a) 3 Gew.-% bis 75 Gew.-%, bevorzugt 5 Gew.-% bis 50 Gew.-%, besonders bevorzugt 10 Gew.-% bis 30 Gew.-% Ölphase,
b) 20 Gew.-% bis 93,5 Gew.-%, bevorzugt 40 Gew.-% bis 89,2 Gew.-%, besonders bevorzugt 60 Gew.-% bis 82,5 Gew.-% Wasserphase,
c) 0,5 Gew.-% bis 10,0 Gew.-%, bevorzugt 1,0 Gew.-% bis 7,0 Gew.-%, besonders bevorzugt 1,5 Gew.-% bis 5,0 Gew.-% Öl-in-Wasser-Emulgator,
d) 0,5 Gew.-% bis 10 Gew.-%, bevorzugt 0,8 Gew.-% bis 8,0 Gew.-%, besonders bevorzugt 1,0 Gew.-% bis 5,0 Gew.-% ein oder mehrere Co-Tenside und
e) 2,5 Gew.-% bis 50 Gew.-%, bevorzugt 4 Gew.-% bis 30 Gew.-%, besonders bevorzugt 5 Gew.-% bis 20 Gew.-% Treibgas
enthält.

6. Verfahren zur Herstellung einer geschäumten, Öl-in-Wasser-Emulsion enthaltenden Formulierung gemäß mindestens einem der Ansprüche 1 bis 5 umfassend die Verfahrensschritte:
A) Bereitstellen einer Öl-in-Wasser-Emulsion enthaltend mindestens ein Co-Tensid in einem geschlossenen Gefäß aufweisend eine Düse
B) Erzeugen eines Überdruckes in dem geschlossenen Gefäß mit Hilfe eines Treibgases
C) Entlassen mindestens eines Teils der Öl-in-Wasser-Emulsion und des Treibgases durch die Düse unter Entstehen einer geschäumten, Öl-in-Wasser-Emulsion enthaltenden Formulierung.

7. Verfahren gemäß Anspruch 6, **dadurch gekennzeichnet, dass** das Treibgas ausgewählt ist aus der Gruppe bestehend aus Luft, CO₂, N₂, N₂O, Propan, Butan, Isobutan und Dimethylether

8. Vorrichtung zur Herstellung einer geschäumten, Öl-in-Wasser-Emulsion enthaltenden Formulierung gemäß mindestens einem der Ansprüche 1 bis 5 umfassend ein geschlossenes Gefäß aufweisend eine Düse, wobei das Gefäß eine Öl-in-Wasser-Emulsion, ein Co-Tensid und ein Treibgas unter Überdruck beherbergt.
